# EUROPÄISCHE PATENTSCHRIFT

(11) **EP 1 452 143 B1**
(45) Veröffentlichungstag und Bekanntmachung des Hinweises auf die Patenterteilung: **21.09.2005**
(21) Anmeldenummer: 04013571.7
(22) Anmeldetag: 27.07.2002
(51) Int. Cl.: A61B 17/32

(54) **Schere, insbesondere für chirurgische Zwecke**
Scissors, especially for surgical purpose
Ciseaux, notamment à usage chirurgical

(30) Priorität: 04.08.2001 DE 10138356
(43) Veröffentlichungstag der Anmeldung: 01.09.2004
(62) Teilanmeldung aus: 02753086.4
(73) Patentinhaber: Aesculap AG & Co. KG, 78532 Tuttlingen (DE)
(72) Erfinder: Dworschak, Manfred, 78589 Dürbheim (DE); Lutze, Theodor, 78582 Balgheim (DE); Morales, Pedro, 78532 Tuttlingen-Nendingen (DE); Weisshaupt, Dieter, 78194 Immendingen (DE)
(74) Vertreter: Hoeger, Stellrecht & Partner Patentanwälte

(56) Entgegenhaltungen:
- DE-A- 3 219 260
- DE-A- 19 733 035
- DE-C- 19 908 367
- DE-U- 29 713 631
- US-A- 3 052 026

## Beschreibung

Die Erfindung betrifft eine Schere, insbesondere für chirurgische Zwecke, mit zwei gegeneinander verschwenkbaren Branchen, die am vorderen Ende jeweils einen Schneidabschnitt mit einer Schneidkante und am hinteren Ende jeweils einen Griffabschnitt aufweisen, wobei mindestens eine der Branchen in ihrem Schneidabschnitt auf ihrer der anderen Branche zugewandten Innenfläche mit einer flächig an ihr anliegenden Metallfolie verbunden ist, die sich zumindest am schneidkantenseitigen Ende der Innenfläche bis an dieses erstreckt oder geringfügig darüber hervorsteht und deren an diesem Ende der Innenfläche angeordnete Kante die Schneidkante dieser Innenfläche bildet.

Die US-A-3052026 zeigt eine solche Schere.

Chirurgische Scheren werden vielfältig eingesetzt, beispielsweise im chirurgischen Bereich zum Schneiden von Gewebe, von Abdecktüchern, von Nähfäden etc., und in allen Fällen ist es wichtig, daß eine wirksame und scharfe Schneidkante zur Verfügung steht. Es ist daher üblich, diese Scheren zu schleifen, dieser Arbeitsgang ist aufwendig und vom Gelingen dieses Arbeitsganges ist abhängig, ob die Schere für den Einsatzzweck brauchbar ist oder nicht.

Es ist Aufgabe der Erfindung, eine gattungsgemäße Schere so auszubilden, daß sie besonders einfach herstellbar ist, insbesondere ohne die Notwendigkeit eines Schleifvorganges für die Schneidkante.

Diese Aufgabe wird bei einer Schere der eingangs beschriebenen Art erfindungsgemäß dadurch gelöst, daß die Metallfolie eine Dicke zwischen 0,05 und 0,4 mm aufweist und daß die Metallfolie mit der Innenfläche der Branche verklebt ist.

Die Metallfolie kann aus verschiedenen Metallen hergestellt werden, besonders vorteilhaft ist die Verwendung einer Metallfolie aus Federstahl.

Außerdem ist es günstig, wenn die Metallfolie aus einem Metall mit einer hohen Härte besteht, beispielsweise einer Härte von mindestens HRC60.

Die Metallfolie kann auf verschiedene Weise hergestellt sein, beispielsweise kann die Metallfolie ein Stanzteil sein oder ein Laserschneidteil oder ein Drahterodierteil. In allen Fällen wird die Metallfolie aus einer größeren flächigen Metallfolie herausgearbeitet und kann dann nach diesem Herausarbeiten beispielsweise durch Stanzen, Laserschneiden oder Drahterodieren unmittelbar verwendet werden, eine Nachbearbeitung ist nicht mehr notwendig.

Zur Verklebung von Branche und Metallfolie kann zwischen Metallfolie und Innenfläche ein thermisch auspolymerisierbarer Klebstoff angeordnet sein oder ein Heißschmelzkleber.

Besonders vorteilhaft ist es dabei, wenn zwischen Metallfolie und Innenfläche eine doppelseitige Klebefläche angeordnet ist, beispielsweise ein doppelseitiges Klebeband, wie es zum Festlegen von Bremsscheiben in der Automobilindustrie Verwendung findet. Es ist auch möglich, die Metallfolie in anderer Weise mit der Innenfläche der Branche zu verbinden, beispielsweise kann die Metallfolie mit der Branche verschweißt oder verlötet sein, bei der Verwendung von Kunststoff als Material der Branche kann die Metallfolie auch in den Kunststoff eingespritzt sein. Dabei ist es vorteilhaft, wenn die Folienoberfläche auf der der Innenfläche zugewandten Seite strukturiert ist, so daß in diesem Bereich eine besonders innige Verbindung zwischen dem Material der Branche und der Metallfolie erreicht wird.

Insbesondere kann die Metallfolie auf ihrer der Innenfläche der Branche zugewandten Seite Vorsprünge tragen, die in das Material der Branche eintauchen, vorzugsweise weisen die Vorsprünge dazu Hinterschnitte auf.

Die Struktur der Vorsprünge kann beispielsweise blumenkohlähnlich sein, dabei sind die Vorsprünge über die Metallfolie unregelmäßig verteilt und bilden kugelige oder annähernd kugelige Vorsprünge aus, die über stielartige Verbindungen mit der Metallfolie verbunden sind.

Derartige Vorsprünge können beispielsweise durch eine elektrolytisch auf die Folie aufgebrachte Haftschicht ausgebildet werden.

Bei einer besonders bevorzugten Ausführungsform bestehen die Vorsprünge aus Nickel.

Es ist auch möglich, die Folienoberfläche auf der der Innenfläche zugewandten Seite dadurch zu strukturieren, insbesondere Vorsprünge an dieser Seite zu erzeugen, daß die Metallfolie einer Ätzbehandlung unterworfen wird.

Besonders vorteilhaft ist es, wenn die Metallfolie und die Innenfläche der Branche durch Heißprägung miteinander verbunden sind. Bei einer solchen Heißprägung werden die Metallfolie und die Branche unter Druck und gegebenenfalls zusätzliche Erwärmung gegeneinandergepreßt, dabei dringen die Vorsprünge in das Kunststoffmaterial der Branche ein, und dieses Kunststoffmaterial kann die Vorsprünge umfließen, so daß sich nach der Abkühlung eine verzahnte Verbindung zwischen Metallfolie und Branche ergibt.

Die Schneidkante der Metallfolie kann glatt ausgebildet sein, gemäß einer bevorzugten Ausführungsform ist jedoch vorgesehen, daß die die Schneidkante der Metallfolie bildende Kante nebeneinanderliegende Vor- und Rücksprünge aufweist, insbesondere zahnförmige Vor- und Rücksprünge. Es ergibt sich dadurch eine ähnliche Wirkung wie bei einem Wellenschliff, das Abgleiten des zwischen den Schneidkanten angeordneten und zu schneidenden Teils wird dadurch verhindert.

Bei einer bevorzugten Ausführungsform kann weiterhin vorgesehen sein, daß die Innenfläche in Richtung auf die gegenüberliegende Branche erhabene Vorsprünge trägt, die in Ausschnitte der Metallfolie eingreifen und diese dadurch auf der Innenfläche positionieren. Diese Positionierung dient einmal der Fertigung, denn es ist auf diese Weise sehr einfach, die Metallfolie relativ zur Innenfläche in die Lage zu bringen, in der eine Verbindung mit der Innenfläche erfolgen soll, zum anderen wird dadurch aber auch die Metallfolie relativ zur Innenfläche stabilisiert, so daß die Verbindung zwischen Metallfolie und Innenfläche, beispielsweise die Klebeverbindung, weniger stark belastet wird, wenn beim Schneiden Scherkräfte auf die Metallfolie einwirken.

Ein Vorsprung kann beispielsweise an der der Schneidkante gegenüberliegenden Kante der Innenfläche angeordnet sein, dabei ist es vorteilhaft, wenn sich der Vorsprung parallel zur Kante der Innenfläche über einen Teil der Länge dieser Kante erstreckt.

Es ist auch möglich, daß ein Vorsprung am griffabschnittseitigen Ende der Innenfläche angeordnet ist, dann ist es vorteilhaft, wenn dieser Vorsprung in Richtung auf das vordere Ende der Innenfläche konvergiert, insbesondere keilförmig ausgebildet ist. Ein solcher Vorsprung wirkt als Zentrierung, so daß die auf die Innenfläche aufgelegte Metallfolie beim Auflegen direkt und exakt in die gewünschte Position geschoben wird.

Es ist vorteilhaft, wenn die Innenflächen konkav geformt und die Schneidabschnitte der beiden Branchen federnd gegeneinander gedrückt sind. Dadurch liegen die Schneidkanten an der eigentlichen Schnittstelle immer zuverlässig aneinander an und gewährleisten dadurch einen einwandfreien Schnitt.

Außerdem kann vorgesehen sein, daß die Schneidabschnitte eine Verschränkung aufweisen. Durch eine solche Verschränkung werden die Schneidabschnitte um ihre Längsachse geringfügig gegeneinander verschwenkt, so daß sie einen kleinen Winkel miteinander einschließen, dadurch liegen die Innenflächen nicht flächig aneinander, sondern erzeugen eine Art Freiwinkel, wie er bei üblichen Scheren durch einen bestimmten Schliff der Schneidkanten erreicht werden kann.

Grundsätzlich kann die beschriebene Konstruktion einer Schere bei Branchen mit unterschiedlichen Materialien eingesetzt werden, also auch bei Metallscheren, besonders vorteilhaft ist eine solche Ausgestaltung aber bei einer Schere, deren Branchen aus Kunststoff bestehen. Auf diese Weise können Scheren hergestellt werden, die als Branchenmaterial Kunststoffe verwenden, die selbst keine geeigneten Schneidkanten ausbilden können, durch Auflegen der beschriebenen Metallfolien auf die Innenflächen ist es jedoch möglich, Scheren herzustellen, die fast vollständig aus Kunststoff bestehen und trotzdem hervorragende Schneideigenschaften aufweisen.

Insbesondere im chirurgischen Bereich können als Kunststoffe beispielsweise Polyamide (PA, PPA), Polyetheretherketon (PEEK) oder Flüssigkristallpolymere (LCP) verwendet werden, günstig ist es dabei, wenn der Kunststoff mit Fasern verstärkt ist, beispielsweise mit Glasfasern oder mit Karbonfasern.

Die Innenflächen der Branchen mit der Metallfolie können sich in Richtung auf ihre Griffabschnitte bis über die Schwenkverbindung der Branchen erstrecken, insbesondere kann die Schwenkverbindung im Mittelteil der mit der Metallfolie versehenen Innenflächen angeordnet sein, so daß die Schwenkverbindung die beiden Innenflächen federnd gegeneinanderdrücken kann.

Dabei sind die unterschiedlichsten Schwenkverbindungen möglich, besonders vorteilhaft sind Schwenkverbindungen, die durch einfaches formschlüssiges Zusammenfügen der beiden Branchen hergestellt werden können, so daß keine zusätzlichen Wellenelemente Verwendung finden müssen, es ist aber auch ohne weiteres möglich, die beschriebene Ausgestaltung bei Scheren zu verwenden, deren Branchen über Wellen, Schrauben etc. schwenkbar miteinander verbunden sind.

Die nachfolgende Beschreibung bevorzugter Ausführungsformen der Erfindung dient im Zusammenhang mit der Zeichnung der näheren Erläuterung. Es zeigen:
- Figur 1:: eine Draufsicht auf eine Schere mit einem Lappenschluß;
- Figur 2:: eine Seitenansicht dieser Schere in Richtung des Pfeiles A in Figur 1;
- Figur 3:: eine Schnittansicht längs Linie 3-3 in Figur 2;
- Figur 4:: einen vergrößerten Ausschnitt gemäß Bereich B in Figur 3 mit einer glatten Schneidenkante;
- Figur 5: eine Ansicht ähnlich Figur 4 mit sägezahnförmiger Schneidenkante;
- Figur 6:: eine Schnittansicht längs Linie 6-6 in Figur 1;
- Figur 7:: eine Draufsicht auf eine strukturierte Oberfläche einer Metallfolie und
- Figur 8:: eine Schnittansicht längs Linie 8-8 in Figur 7.

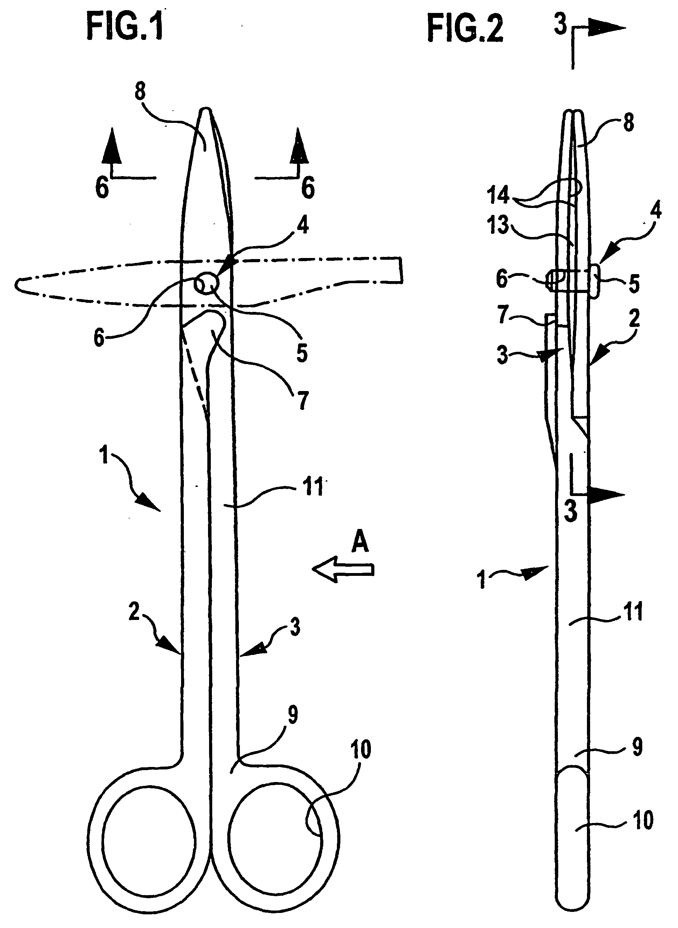

Die in der Zeichnung dargestellte Schere 1 umfaßt zwei Branchen 2, 3, die an einem Schwenklager 4 verschwenkbar miteinander verbunden sind. Dieses Schwenklager wird bei dem dargestellten Ausführungsbeispiel gebildet durch einen eine Branche 2 durchsetzenden Lagerstift 5, der von dieser Branche 2 in Richtung auf die andere Branche 3 absteht. Die andere Branche 3 weist in diesem Bereich eine Lageröffnung 6 auf, in die der Lagerstift 5 eingreift. Zur axialen Festlegung der Branche 3 auf dem Lagerstift 5 ist an der Branche 2 ein seitlich abstehender Lappen 7 angeordnet, der die Branche 3 übergreift, wenn beide Branchen 2, 3 parallel zueinander in der Schließstellung stehen, wie es in Figur 1 mit ausgezogenen Linien dargestellt ist. Wenn die Branche 3 dagegen um etwa 90° gegenüber dieser Schließstellung verschwenkt ist (in Figur 1 in strichpunktierten Linien dargestellt), dann taucht sie nicht mehr unter den Lappen 7 ein und kann ohne weiteres vom Lagerstift 5 abgenommen werden. Es ist damit eine werkzeugfreie Montage der beiden Branchen 2, 3 möglich, wobei die Branchen 2, 3 in der normalen Arbeitsstellung durch den Lappen 7 in axialer Richtung relativ zueinander fixiert sind.

Bei dem in der Zeichnung dargestellten Ausführungsbeispiel ist der Lagerstift 5 als separates Bauteil ausgebildet, er kann aber auch einstückig mit der Branche 2 ausgebildet sein.

Jede der beiden Branchen 2, 3 weist einen Schneidabschnitt 8 auf, der am vorderen Ende der Branchen 2, 3 angeordnet ist, einen Griffabschnitt 9 mit Fingerösen 10 am gegenüberliegenden Ende und einen Verbindungsbereich 11 zwischen Schneidabschnitt 8 und Griffabschnitt 9.

Im Schneidabschnitt 8 sind beide Branchen 2, 3 mit einer jeweils zur anderen Branche weisenden Innenfläche 12 versehen, diese Innenfläche 12 ist geringfügig konkav ausgebildet, so daß zwischen den beiden Innenflächen 12 der einander gegenüberliegenden Branchen 2, 3 ein schmaler Schlitz 13 entsteht (Figur 2). Das Schwenklager 4 befindet sich etwa in der Mitte der Längsausdehnung dieser Innenflächen 12, und die Schneidabschnitte 8 der beiden Branchen 2, 3 werden durch das Schwenklager 4 bzw. den Lappen 7 elastisch gegeneinander gedrückt, so daß dadurch die Breite des Schlitzes 13 reduziert wird.

Bei dem in der Zeichnung dargestellten Ausführungsbeispiel sind die beiden Branchen 2 und 3 jeweils einstückig ausgebildet und bestehen aus Kunststoff, beispielsweise aus Polyamid (PA, PPA) aus Polyetheretherketon (PEEK) oder aus einem Flüssigkristallpolymer (LCP), vorzugsweise ist dieser Kunststoff durch Einbettung von faserförmigen Werkstoffen verstärkt, beispielsweise von Glasfasern oder Kohlefasern. Es ist bei dieser Ausgestaltung einfach, an der Branche 2 den Lagerstift 5 und an der Branche 3 den Lappen 7 einstückig anzuformen, so daß insgesamt die Schere nur aus zwei Teilen aufgebaut ist.

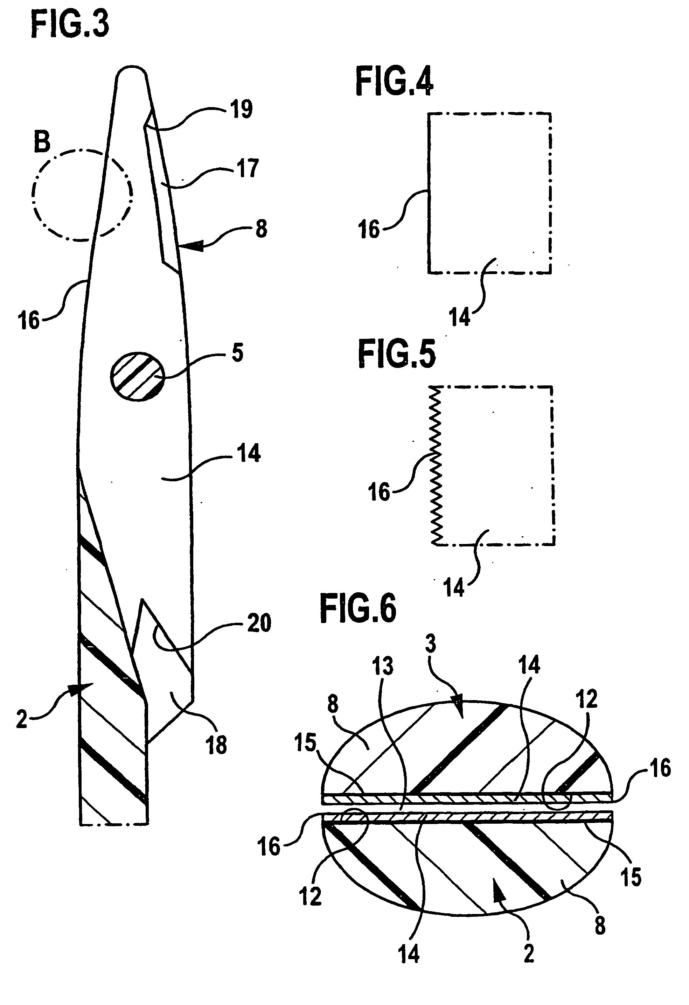

Auf jede der beiden Innenflächen 12 ist flächig eine Metallfolie 14 aufgelegt, diese Metallfolie 14 ist mit der Innenfläche 12 verklebt, beispielsweise durch Zwischenlage eines doppelseitigen Klebebandes 15 (Figur 6), welches mit einem durch Wärme auspolymerisierenden Klebstoff beschichtet sein kann.

Die Metallfolie 14 besteht aus einem Metall hoher Härte, insbesondere mit einer Härte von mindestens HRC60, es ist günstig, als Metall einen Federstahl zu verwenden. Die Dicke der Metallfolie 14 liegt dabei zwischen 0,05 und 0,4 mm. Die Form der Metallfolie 14 entspricht der Form der Innenfläche 12, die Metallfolie fluchtet mit der Seitenkante der Innenfläche 12 oder steht allenfalls ganz geringfügig darüber hervor, wobei die beim Öffnen der Branchen 2, 3 aneinander entlang gleitenden Kanten der Metallfolien 14 die Schneidkanten 16 der Schere 1 ausbilden.

Die Metallfolien 14 sind aus einer größeren Metallfolie herausgearbeitet, beispielsweise durch Ausstanzen, durch Drahterodieren oder durch Laserschneiden, und können ohne weitere Bearbeitung auf die Innenflächen 12 aufgelegt und mit diesen durch Verkleben verbunden werden. Um eine gute Positionierung der Metallfolien 14 zu erreichen, weisen die Innenflächen 12 erhabene Vorsprünge 17, 18 auf, die in Richtung auf die Innenfläche der gegenüberliegenden Branche vorstehen und die in komplementäre Ausnehmungen 19 bzw. 20 der Metallfolie 14 eingreifen. Ein Vorsprung 17 läuft an der der Schneidkante 16 gegenüberliegenden Kante der Innenfläche 12 entlang und erstreckt sich über einen Teil der Länge dieser Kante, der andere Vorsprung 18 ist an dem griffabschnittseitigen Ende der Innenfläche 12 angeordnet und in Richtung auf das vordere Ende der Schere 1 keilförmig ausgebildet, so daß sich beim Auflegen der Metallfolie 14 auf die Innenfläche 12 bereits durch die Vorsprünge 17 und 18 eine exakte Ausrichtung der Metallfolie relativ zur Innenfläche 12 ergibt. In dieser Lage kann dann die Verklebung erfolgen.

Die von der Metallfolie 14 ausgebildete Schneidkante 16 kann glatt ausgebildet sein (Figur 4) oder aber in einem abgewandelten Ausführungsbeispiel zahnförmig (Figur 5), so daß dadurch das zu schneidende Gut zwischen den Schneidkanten 16 gehalten und gegen ein Abrutschen gesichert wird.

Es ist günstig, wenn die Verklebung der Metallfolie 14 mit der Innenfläche 12 mittels eines Klebers erfolgt, der durch Wärme aktivierbar ist, auf diese Weise ist es bei Branchen, die aus thermoplastischem Kunststoff hergestellt werden, möglich, die Verklebung beim Herstellungsprozeß der Branchen oder unmittelbar danach vorzunehmen, wobei die Bildungswärme der Branchen zur Aktivierung des Klebstoffes verwendet wird.

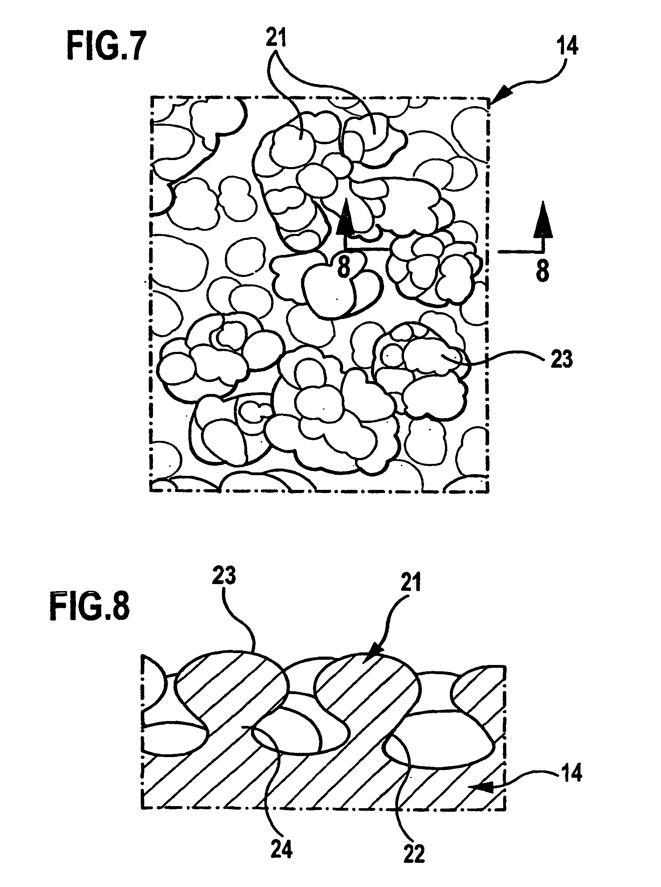

Die Verbindung zwischen Metallfolie und der Innenfläche der Branche kann noch dadurch verbessert werden, daß die Metallfolie 14 auf ihrer der Innenfläche 12 der Branchen 2, 3 zugewandten Seite Vorsprünge 21 trägt, die vorzugsweise Hinterschnitte 22 aufweisen. Es handelt sich dabei in der Regel um eine Mikrostruktur, die in den Figuren 7 und 8 sehr vergrößert dargestellt ist. Die Vorsprünge können völlig unregelmäßig über die Oberfläche der Metallfolie 14 verteilt sein, beispielsweise kann die Struktur blumenkohlähnlich sein, d.h. die Vorsprünge 21 haben eine kugelige oder allgemein konvexe Außenfläche 21 und sind über eine stielartige Verbindung 24 mit der Metallfolie 14 verbunden. Diese Oberflächenstruktur kann beispielsweise durch einen Ätzvorgang erzeugt werden oder besonders vorteilhaft durch eine elektrolytische Abscheidung einer Haftschicht auf der Metallfolie, insbesondere einer Haftschicht aus Nickel.

Zur Verbindung mit der Branche 2, 3 wird die in dieser Weise oberflächlich strukturierte Metallfolie 14 flächig gegen die Innenseite 12 der Branchen 2, 3 gedrückt, gegebenenfalls unter zusätzlicher Erwärmung, dabei fließt das Kunststoffmaterial der Branchen 2, 3 und verzahnt sich mit den Vorsprüngen 21 und deren Hinterschnitten 22. Dadurch erhält man nach der Abkühlung eine flächige und über die gesamte Oberfläche gleichmäßig dauerhafte Verbindung zwischen Metallfolie 14 und den Innenflächen 12 der Branchen 2, 3.

## Patentansprüche

1. Schere, insbesondere für chirurgische Zwecke, mit zwei gegeneinander verschwenkbaren Branchen (2, 3), die am vorderen Ende jeweils einen Schneidabschnitt (8) mit einer Schneidkante (16) und am hinteren Ende jeweils einen Griffabschnitt (9) aufweisen, wobei mindestens eine der Branchen (2, 3) in ihrem Schneidabschnitt (8) auf ihrer der anderen Branche zugewandten Innenfläche (12) mit einer flächig an ihr anliegenden Metallfolie (14) verbunden ist, die sich zumindest am schneidkantenseitigen Ende der Innenfläche (12) bis an dieses erstreckt oder geringfügig darüber hervorsteht und deren an diesem Ende der Innenfläche (12) angeordnete Kante die Schneidkante (16) dieser Innenfläche (12) bildet, **dadurch gekennzeichnet, daß** die Metallfolie (14) eine Dicke zwischen 0,05 und 0,4 mm aufweist und daß die Metallfolie (14) mit der Innenfläche (12) der Branche (2, 3) verklebt ist.

2. Schere nach Anspruch 1, **dadurch gekennzeichnet, daß** die Metallfolie (14) aus Federstahl besteht.

3. Schere nach einem der voranstehenden Ansprüche, **dadurch gekennzeichnet, daß** die Metallfolie (14) aus einem Metall mit einer hohen Härte besteht.

4. Schere nach einem der voranstehenden Ansprüche, **dadurch gekennzeichnet, daß** die Metallfolie (14) ein Stanzteil ist.

5. Schere nach einem der Ansprüche 1 bis 3, **dadurch gekennzeichnet, daß** die Metallfolie (14) ein Laserschneidteil ist.

6. Schere nach einem der voranstehenden Ansprüche 1 bis 3, **dadurch gekennzeichnet, daß** die Metallfolie (14) ein Drahterodierteil ist.

7. Schere nach einem der voranstehenden Ansprüche, **dadurch gekennzeichnet, daß** zwischen Metallfolie (14) und Innenfläche (12) ein thermisch auspolymerisierbarer Klebstoff angeordnet ist.

8. Schere nach einem der Ansprüche 1 bis 6, **dadurch gekennzeichnet, daß** zwischen Metallfolie (14) und Innenfläche (12) ein Heißschmelzkleber angeordnet ist.

9. Schere nach einem der voranstehenden Ansprüche, **dadurch gekennzeichnet, daß** zwischen Metallfolie (14) und Innenfläche (12) eine doppelseitige Klebefläche (15) angeordnet ist.

10. Schere nach einem der voranstehenden Ansprüche, **dadurch gekennzeichnet, daß** die Metallfolie (14) auf ihrer der Innenfläche (12) der Branche (2, 3) zugewandten Seite Vorsprünge (21) trägt, die in das Material der Branche (2, 3) eintauchen.

11. Schere nach Anspruch 10, **dadurch gekennzeichnet, daß** die Vorsprünge (21) Hinterschnitte (22) aufweisen.

12. Schere nach einem der Ansprüche 10 oder 11, **dadurch gekennzeichnet, daß** die Vorsprünge (21) über die Metallfolie (14) unregelmäßig verteilt sind.

13. Schere nach einem der Ansprüche 10 bis 12, **dadurch gekennzeichnet, daß** die Vorsprünge (21) durch eine elektrolytisch auf die Metallfolie (14) aufgebrachte Haftschicht ausgebildet werden.

14. Schere nach einem der Ansprüche 10 bis 13, **dadurch gekennzeichnet, daß** die Vorsprünge (21) aus Nickel bestehen.

15. Schere nach einem der Ansprüche 10 bis 14, **dadurch gekennzeichnet, daß** die Vorsprünge (21) durch eine Ätzbehandlung der Metallfolie (14) gebildet werden.

16. Schere nach einem der Ansprüche 10 bis 15, **dadurch gekennzeichnet, daß** die Metallfolie (14) und die Innenfläche (12) der Branche (2, 3) durch Heißprägung miteinander verbunden sind.

17. Schere nach einem der voranstehenden Ansprüche, **dadurch gekennzeichnet, daß** die die Schneidkante (16) der Metallfolie (14) bildende Kante nebeneinanderliegende Vor- und Rücksprünge aufweist.

18. Schere nach Anspruch 17, **dadurch gekennzeichnet, daß** die Vor- und Rücksprünge zahnförmig sind.

19. Schere nach einem der voranstehenden Ansprüche, **dadurch gekennzeichnet, daß** die Innenfläche (12) in Richtung auf die gegenüberliegende Branche (2, 3) erhabene Vorsprünge (17, 18) trägt, die in Ausschnitte (19 bzw. 20) der Metallfolie (14) eingreifen und diese **dadurch** auf der Innenfläche (12) positionieren.

20. Schere nach Anspruch 19, **dadurch gekennzeichnet, daß** ein Vorsprung (17) an der der Schneidkante (16) gegenüberliegenden Kante der Innenfläche (12) angeordnet ist.

21. Schere nach Anspruch 20, **dadurch gekennzeichnet, daß** sich der Vorsprung (17) parallel zur Kante der Innenfläche (12) über einen Teil der Länge dieser Kante erstreckt.

22. Schere nach einem der Ansprüche 19 bis 21, **dadurch gekennzeichnet, daß** ein Vorsprung (18) am griffabschnittseitigen Ende der Innenfläche (12) angeordnet ist.

23. Schere nach Anspruch 22, **dadurch gekennzeichnet, daß** dieser Vorsprung in Richtung auf das vordere Ende der Innenfläche (12) konvergiert.

24. Schere nach Anspruch 23, **dadurch gekennzeichnet, daß** der Vorsprung (18) keilförmig ausgebildet ist.

25. Schere nach einem der voranstehenden Ansprüche, **dadurch gekennzeichnet, daß** die Innenflächen (12) konkav geformt und die Schneidabschnitte (8) der beiden Branchen (2, 3) federnd gegeneinander gedrückt sind.

26. Schere nach einem der voranstehenden Ansprüche, **dadurch gekennzeichnet, daß** die Schneidabschnitte (8) eine Verschränkung aufweisen.

27. Schere nach einem der voranstehenden Ansprüche, **dadurch gekennzeichnet, daß** die Branchen (2, 3) aus Kunststoff bestehen.

28. Schere nach Anspruch 27, **dadurch gekennzeichnet, daß** der Kunststoff mit Fasern verstärkt ist.

29. Schere nach einem der voranstehenden Ansprüche, **dadurch gekennzeichnet, daß** sich die Innenflächen (12) der Branchen (2, 3) mit der Metallfolie (14) in Richtung auf ihre Griffabschnitte (9) bis über die Schwenkverbindung (4) der Branchen (2, 3) erstrecken.

30. Schere nach Anspruch 29, **dadurch gekennzeichnet, daß** die Schwenkverbindung (4) im Mittelteil der mit der Metallfolie (14) versehenen Innenflächen (12) angeordnet ist.

## Claims

1. Scissors, in particular for surgical purposes, with two arms (2, 3) which are pivotable relative to each other and which in each case have a cutting portion (8) with a cutting edge (16) at the front end and a handle portion (9) at the rear end, wherein at least one of the arms (2, 3) is joined on the inner face (12) of its cutting portion (8) facing the other arm to a metallic foil (14) which rests flat against it and which at least at the end of the inner face (12) towards the cutting edge extends as far as the said end of the inner face (12) or projects slightly beyond it and of which the edge situated at this end of the inner face (12) forms the cutting edge (16) of the said inner face (12), **characterized in that** the metallic foil (14) has a thickness of between 0.05 and 0.4 mm, and **in that** the metallic foil (14) is adhesively joined to the inner face (12) of the arm (2, 3).

2. Scissors according to Claim 1, **characterized in that** the metallic foil (14) consists of spring steel.

3. Scissors according to one of the preceding Claims, **characterized in that** the metallic foil (14) consists of a metal with a high degree of hardness.

4. Scissors according to one of the preceding Claims, **characterized in that** the metallic foil (14) is a part formed by stamping.

5. Scissors according to one of Claims 1 to 3, **characterized in that** the metallic foil (14) is a part formed by laser-cutting.

6. Scissors according to one of the preceding Claims 1 to 3, **characterized in that** the metallic foil (14) is a part formed by wire-erosion.

7. Scissors according to one of the preceding Claims, **characterized in that** an adhesive capable of being thermally polymerized out is arranged between metallic foil (14) and inner face (12).

8. Scissors according to one of Claims 1 to 6, **characterized in that** a hotmelt adhesive is arranged between metallic foil (14) and inner face (12).

9. Scissors according to one of the preceding Claims, **characterized in that** a double-sided adhesive face (15) is arranged between metallic foil (14) and inner face (12).

10. Scissors according to one of the preceding Claims, **characterized in that** on its side facing the inner face (12) of the arm (2, 3) the metallic foil (14) comprises projections (21) which project into the material of the arm (2, 3).

11. Scissors according to Claim 10, **characterized in that** the projections (21) have undercuts (22).

12. Scissors according to one of Claims 10 or 11, **characterized in that** the projections (21) are distributed irregularly over the metallic foil (14).

13. Scissors according to one of Claims 10 to 12, **characterized in that** the projections (21) are formed by an adhesive layer applied electrolytically to the metallic foil (14).

14. Scissors according to one of Claims 10 to 13, **characterized in that** the projections (21) consist of nickel.

15. Scissors according to one of Claims 10 to 14, **characterized in that** the projections (21) are formed by an etching treatment of the metallic foil (14).

16. Scissors according to one of Claims 10 to 15, **characterized in that** the metallic foil (14) and the inner face (12) of the arm (2, 3) are joined together by hot stamping.

17. Scissors according to one of the preceding Claims, **characterized in that** the edge forming the cutting edge (16) of the metallic foil (14) has projections and recesses arranged adjacent to one another.

18. Scissors according to Claim 17, **characterized in that** the projections and recesses are toothed-shaped.

19. Scissors according to one of the preceding Claims, **characterized in that** the inner face (12) comprises projections (17, 18) which are raised in the direction towards the opposite arm (2, 3) and which engage in cut-away portions (19 and 20, respectively) in the metallic foil (14) and thereby position the latter on the inner face (12).

20. Scissors according to Claim 19, **characterized in that** a projection (17) is arranged on the edge of the inner face (12) opposite the cutting edge (16).

21. Scissors according to Claim 20, **characterized in that** the projection (17) extends parallel to the edge of the inner face (12) over part of the length of the said edge.

22. Scissors according to one of Claims 19 to 21, **characterized in that** a projection (18) is arranged at the end of the inner face (12) towards the handle portion.

23. Scissors according to Claim 22, **characterized in that** the said projection converges in the direction towards the front end of the inner face (12).

24. Scissors according to Claim 23, **characterized in that** the projection (18) is wedge-shaped.

25. Scissors according to one of the preceding Claims, **characterized in that** the inner faces (12) are shaped in a concave manner and the cutting portions (8) of the two arms (2, 3) are pressed against each other in a resilient manner.

26. Scissors according to one of the preceding Claims, **characterized in that** the cutting portions (8) have a curvature.

27. Scissors according to one of the preceding Claims, **characterized in that** the arms (2, 3) consist of plastics material.

28. Scissors according to Claim 27, **characterized in that** the plastics material is reinforced with fibres.

29. Scissors according to one of the preceding Claims, **characterized in that** the inner faces (12) of the arms (2, 3) with the metallic foil (14) extend in the direction towards their handle portions (9) to beyond the pivoting connection (4) of the arms (2, 3).

30. Scissors according to Claim 29, **characterized in that** the pivoting connection (4) is arranged in the middle part of the inner faces (12) provided with the metallic foil (14).

## Revendications

1. Ciseaux, notamment à usage chirurgical, comportant deux branches (2, 3) pouvant pivoter l'une par rapport à l'autre et qui possèdent sur l'extrémité avant respectivement une section de coupe (8) comportant une arête de coupe (16) et, sur l'extrémité arrière, respectivement une section de saisie (9), dans lesquels au moins l'une des branches (2, 3) est reliée, dans sa section de coupe (8) sur sa surface intérieure (12) tournée vers l'autre branche, à une feuille métallique (14) appliquée à plat sur sa surface et qui s'étend au moins sur l'extrémité, située du côté de l'arête de coupe, de la surface intérieure (12) jusqu'à cette extrémité ou fait saillie légèrement par rapport à cette dernière, et dont l'arête, disposée sur cette extrémité de la surface intérieure (12), forme l'arête de coupe (16) de cette surface intérieure (12), **caractérisés en ce que** la feuille métallique (14) possède une épaisseur entre 0,05 et 0,4 mm et que la feuille métallique (14) est collée à la surface intérieure (12) des branches (2, 3).

2. Ciseaux selon la revendication 1, **caractérisés en ce que** la feuille métallique (14) est réalisée en un acier poreux pour ressorts.

3. Ciseaux selon l'une des revendications précédentes, **caractérisés en ce que** la feuille métallique (14) est formée d'un métal possédant une dureté élevée.

4. Ciseaux selon l'une des revendications précédentes, **caractérisée en ce que** la feuille métallique (14) est une pièce découpée.

5. Ciseaux selon l'une des revendications 1 à 3, **caractérisés en ce que** la feuille métallique (14) est une pièce découpée par laser.

6. Ciseaux selon l'une des revendications précédentes 1 à 3, **caractérisés en ce que** la feuille métallique (14) est une pièce obtenue par érosion avec un fil.

7. Ciseaux selon l'une des revendications précédentes, **caractérisés en ce qu'**un adhésif autopolymérisable thermiquement est disposé entre la feuille métallique (14) et la surface intérieure (12).

8. Ciseaux selon l'une des revendications 1 à 6, **caractérisés en ce qu'**une colle fusible à chaud est disposée entre la feuille métallique (14) et la surface intérieure (12).

9. Ciseaux selon l'une des revendications précédentes, **caractérisés en ce qu'**une couche adhésive double face (15) est disposée entre la feuille métallique (14) et la surface intérieure (12).

10. Ciseaux selon l'une des revendications précédentes, **caractérisés en ce que** la feuille métallique (14) porte, sur son côté tourné vers la surface intérieure (12) de la branche (2, 3), des parties saillantes (21), qui pénètrent dans le matériau des branches (2, 3).

11. Ciseaux selon la revendication 10, **caractérisés en ce que** ces parties saillantes (21) possèdent des parties en contre-dépouille (22).

12. Ciseaux selon l'une des revendications 10 ou 11, **caractérisés en ce que** les parties saillantes (21) sont réparties de façon irrégulière sur la feuille métallique (14).

13. Ciseaux selon l'une des revendications 10 à 12, **caractérisés en ce que** les parties saillantes (21) sont formées par une couche adhésive déposée par voie électrolytique sur la feuille métallique (14).

14. Ciseaux selon l'une des revendications 10 à 13, **caractérisés en ce que** les parties saillantes (21) sont réalisées en nickel.

15. Ciseaux selon l'une des revendications 10 à 14, **caractérisés en ce que** les parties saillantes (21) sont formées par un traitement d'attaque chimique de la feuille métallique (14).

16. Ciseaux selon l'une des revendications 10 à 15, **caractérisés en ce que** la feuille métallique (14) et la surface intérieure (12) des branches (2, 3) sont reliées entre elles par gaufrage à chaud.

17. Ciseaux selon l'une des revendications précédentes, **caractérisés en ce que** l'arête, qui forme l'arête de coupe (16) de la feuille métallique (14), possède des appendices saillants et des parties rentrantes disposés côte-à-côte.

18. Ciseaux selon la revendication 17, **caractérisés en ce que** les appendices saillantes et les parties rentrantes sont en forme de dents.

19. Ciseaux selon l'une des revendications précédentes, **caractérisés en ce que** la surface intérieure (12) comporte des parties saillantes (17, 18), qui sont surélevées en direction de la branche opposée (2, 3), s'engagent dans des découpes (19 ou 20) de la feuille métallique (14) et positionnent de ce fait cette feuille sur la surface intérieure (12).

20. Ciseaux selon la revendication 19, **caractérisés en ce qu'**une partie saillante (17) est prévue sur le bord de la surface intérieure (12), qui est situé à l'opposé de l'arête de coupe (16).

21. Ciseaux selon la revendication 20, **caractérisés en ce que** la partie saillante (17) s'étend parallèlement au bord de la surface intérieure (12) sur une partie de la longueur de ce bord.

22. Ciseaux selon l'une des revendications 19 à 21, **caractérisés en ce qu'**une partie saillante (18) est disposée sur l'extrémité, côté section de saisie, de la surface intérieure (12).

23. Ciseaux selon la revendication 22, **caractérisés en ce que** cette partie saillante converge en direction de l'extrémité avant de la surface intérieure (12).

24. Ciseaux selon la revendication 23, **caractérisés en ce que** la partie saillante (18) est agencée avec une forme de coin.

25. Ciseaux selon l'une des revendications précédentes, **caractérisés en ce que** les surfaces intérieures (12) ont une configuration concave et les sections de coupe (8) des deux branches (2, 3) sont repoussées élastiquement l'une contre l'autre.

26. Ciseaux selon l'une des revendications précédentes, **caractérisés en ce que** les arêtes de coupe (8) possèdent un système d'assemblage en crémaillère.

27. Ciseaux selon l'une des revendications précédentes, **caractérisés en ce que** les branches (2, 3) sont réalisées en matière plastique.

28. Ciseaux selon la revendication 27, **caractérisés en ce que** la matière plastique est renforcée par des fibres.

29. Ciseaux selon l'une des revendications précédentes, **caractérisés en ce que** les surfaces intérieures (12) des branches (2, 3) pourvues de la feuille métallique (14) s'étendent en direction de leurs sections de préhension (9) jusqu'au-delà de la liaison pivotante (4) des branches (2, 3).

30. Ciseaux selon la revendication 29, **caractérisés en ce que** la liaison pivotante (4) est disposée dans la partie centrale des surfaces intérieures (12) pourvues de la feuille métallique (14).
